# EUROPEAN PATENT APPLICATION

(11) **EP 3 290 021 A1**
(43) Date of publication of application: **07.03.2018**
(21) Application number: 16187103.3
(22) Date of filing: 02.09.2016
(51) Int. Cl.: A61K 8/49, A61Q 17/04, A61K 8/86

(54) **COMPOSTION FOR TOPICAL APPLICATION COMPRISING A PHOTOCHROMIC AGENT**

(71) Applicant: Unilever N.V., 3013 AL Rotterdam (NL)
(72) Inventor: BANDYOPADHYAY, Subhajit., Mohanpur, Nadia, West Bengal 741525 (IN); GHOSH DASTIDAR, Sudipta., Whitefield Bangalore 560 066 (IN); ROY, Biswajit., Mohanpur, Nadia, West Bengal 741525 (IN); RAHAMAN, Sk. Atiur., Mohanpur, Nadia, West Bengal 741525 (IN)
(74) Representative: Corsten, Michael Allan

(57) **Abstract**

The invention relates to a composition for topical application, said composition containing a photochromic agent, said photochromic agent comprising:
(i) one or more photochromic substances selected from spiropyrans, spirooxazines, diarylethenes, azobenzenes, spironaphthoxazines, naphthopyranes, nitrobenzylpyridines and combinations thereof; and
(ii) an aromatic polyether;
wherein the one or more photochromic substances are non-covalently bound to and/or encapsulated by the aromatic polyether.

The inventors have discovered that the photo-stability of certain photochromic substances can be significantly improved by binding these substances to and/or by encapsulating these substances in aromatic polyethers. The photochromic agents so obtained can suitably be applied in composition for topical application, such as sunscreen formulations.

## Description

### TECHNICAL FIELD OF THE INVENTION

The invention relates to a composition for topical application, said composition containing a photochromic agent, said photochromic agent comprising:
- one or more photochromic substances selected from spiropyrans, spirooxazines, diarylethenes, azobenzenes, spironaphthoxazines, naphthopyranes, nitrobenzylpyridines and combinations thereof; and
- an aromatic polyether;
wherein the one or more photochromic substances are non-covalently bound to and/or encapsulated by the aromatic polyether.

### BACKGROUND OF THE INVENTION

Solar radiation includes ultraviolet (UV) radiation wavelength of which is between 200 nm and 400 nm. Exposure of skin to UV-A (320 to 400 nm) and UV-B (290 to 320 nm) radiation causes various problems like reddening of the skin, localized irritation, sunburn, melanoma and formation of wrinkles. UV radiation is also known to cause damage to hair. Therefore, it is desirable to protect the skin and other keratinous substrates of the human body from the harmful effects of both UV-A and UV-B radiation.

Cosmetic compositions comprising sunscreen agents are used to protect the skin against UV radiation. Sunscreen agents are generally classified as organic sunscreens and inorganic sunscreens.

Inorganic sunscreens are generally inorganic particles which provide protection to the skin by way of blocking the solar radiation. Examples of such sunscreens are zinc oxide and titanium dioxide. Problem with using inorganic sunscreens is that they cannot be used at high concentrations since they give an unnatural whiteness to the skin on which they are applied.

Organic sunscreens are organic compounds which act by absorbing the solar radiation at a particular range of wavelength and dissipating that energy by various mechanisms. Organic sunscreens are classified into UV-A sunscreens and UV-B sunscreens, depending on the wavelength range over which they offer protection. The most commonly used UV-A sunscreen is of the dibenzoylmethane class. UV-B organic sunscreens from the class of cinnamic acid, salicylic acid, and diphenyl acrylic acid are well-known and used in sunscreen compositions. In order to have sunscreen protection over a wide range of wavelengths, sunscreen compositions generally include both a UV-A sunscreen and a UV-B sunscreen.

Photochromic molecules form a special class of photo responsive molecules which are capable of reversible transformation between two forms by the absorption of electromagnetic radiation, where the two forms have different absorption spectra. The ability of certain photochromic molecules to change their spectral properties under the influence of sunlight, especially UV-light, make it possible to prepare smart adaptive sunscreen products. The photochromic molecules will get triggered only when exposed to light. Thus, they can be used as on-demand sunscreen that is activated only when needed.

US6531118 BA (AVON PRODUCTS INC, 2003) describes a topical composition having a reversible visible change in color in response to a change in light comprising: at least one photochromic material; and a vehicle acceptable for topical application to skin or hair.

WO02/078665 A1 (COLOR ACCESS INC) describes a composition for application to skin comprising a cosmetically or pharmaceutically acceptable encapsulated liquid UV-absorbing naphthopyran or naphthoxazine dye, or a combination thereof, in combination with a cosmetically or pharmaceutically acceptable carrier. Encapsulation of the UV-absorbing dye provides a measure of photostability and yet permits the dye to absorb UV.

US6361763 BA (CARROLL GEORGE H, 2002) describes a photochromic tanning lotion for application to the skin of a person to protect the skin from the harmful effects of ultraviolet radiation, and to temporarily impart a color simulating a natural tan, comprising: one or more photochromic compounds mixed in an appropriate amount in the lotion for application to the skin, said photochromic compound undergoing a photochemical transformation from colorless to the color of naturally tanned skin when exposed to ultraviolet radiation, and also forming a UV block, to thereby produce the appearance of a natural tan upon exposure to ultraviolet radiation while at the same time protecting the user from UV radiation.

A drawback associated with the use of photochromic molecules are unwanted side reactions that occur when these molecules are exposed to UV-light.

US2011/0189462 AA (POLYCORE OPTICAL PTE LTD) describes a photochromic dye encapsulated by a dendritic polymer.

### SUMMARY OF THE INVENTION

The inventors have discovered that the photo-stability of photochromic substances can be significantly improved by binding these substances to a suitable polymer and/or by encapsulating these substances in such a suitable polymer. More particularly, the inventors have found that aromatic polyethers can suitably be used to stabilize photochromic substances selected from spiropyrans, spirooxazines, diarylethenes, azobenzenes, spironaphthoxazines, naphthopyranes, nitrobenzylpyridines and combinations thereof. The photochromic agents obtained by combining the aforementioned photochromic substances with aromatic polyethers can suitably be applied in composition for topical application, such as sunscreen formulations.

Accordingly, the invention relates to a composition for topical application, said composition containing a photochromic agent, said photochromic agent comprising:
- one or more photochromic substances selected from spiropyrans, spirooxazines, diarylethenes, azobenzenes, spironaphthoxazines, naphthopyranes, nitrobenzylpyridines and combinations thereof; and
- an aromatic polyether;
wherein the one or more photochromic substances are non-covalently bound to and/or encapsulated by the aromatic polyether.

Due to the binding to and/or encapsulation by the aromatic polyether, degradation of the photochromic substance, e.g. by reaction with reactive oxygen species or by dimerization, is effectively minimized. Although the inventors do not wish to be bound by theory, it is believed that the improved photo-stability of the photochromic substances results from pi-stack interaction between the photochromic substance and the aromatic polyether. "Pi-stack interaction" refers to a noncovalent attractive force between two aromatic rings. In addition to pi-stack interaction also other forms of non-covalent bonds may bind the one or more photochromic substances to the aromatic polyether, e.g. hydrogen bonds.

The photochromic agent of the present invention may be prepared, for instance, by means of host-guest self-assembly and/or by encapsulation.

Thus, the invention further provides a method of preparing a photochromic agent, said method comprising:
- providing a solution comprising (i) one or more photochromic substances selected from spiropyrans, spirooxazines, diarylethenes, azobenzenes, spironaphthoxazines, naphthopyranes, nitrobenzylpyridines and combinations thereof; (ii) an aromatic polyether and (iii) organic solvent; and
- removing solvent from the solution to precipitate the photochromic agent.

### DETAILED DESCRIPTION OF THE INVENTION

Accordingly, a first aspect of the invention relates to a composition for topical application, said composition containing a photochromic agent, said photochromic agent comprising:
(i) one or more photochromic substances selected from spiropyrans, spirooxazines, diarylethenes, azobenzenes, spironaphthoxazines, naphthopyranes, nitrobenzylpyridines and combinations thereof; and
(ii) an aromatic polyether;
wherein the one or more photochromic substances are non-covalently bound to and/or encapsulated by the aromatic polyether.

The phrase "composition for topical application" refers to a composition that can suitably be applied to human skin or hair.

The term "photochromic substance" as used herein refers to a substance that, when exposed to light radiation containing ultraviolet rays, such as sunlight, exhibits a reversible change in color. When the ultraviolet radiation is discontinued, such a photochromic compound will return to its original color or colorless state.

The term "aromatic polyether" as used herein refers to a compound comprising an aromatic group and two or more ether groups.

The term "dendritic polymer" as used herein refers to polymers with a densely branched structure. Dendrimers, dendrigraft polymers and hyperbranched polymers are examples of dendritic polymers. Dendritic polymers, including dendrimers and hyperbranched polymers, are prepared by condensation reactions of monomeric units having at least two reactive groups after attachment.

Dendrimers are composed of a plurality of dendrons that emanate from a common core which can be a single atom or a group of atoms. Each dendron generally consists of terminal surface groups, interior branch junctures having branching functionalities greater than or equal to two, and divalent connectors that covalently connect neighboring branching junctures. Dendrons and dendrimers can he prepared by convergent or divergent synthesis. Dendrimers are classified by generation, which refers to the number of repeated branching cycles that are performed during its synthesis. For example, if a dendrimer is made by convergent synthesis, and the branching reactions are performed onto the core molecule three times, the resulting dendrimer is considered a third generation dendrimer.

Hyperbranched polymers represent a class of dendritic polymers which contain high levels of nonideal, irregular branching as compared with the more nearly perfect regular structure of dendrons and dendrimers. Specifically, hyperbranched polymers contain a relatively high number of irregular branching areas in which not every repeat unit contains a branch juncture.

Dendrografts (or dendrigraft polymers) are highly branched molecules synthesized from polymeric chains, typically assembled according to a generation-based scheme analogous to dendrimers, that relies on cycles of substrate functionalization and grafting. In contrast to dendrimers, the coupling sites are distributed randomly on the substrate rather than strictly located at the chain ends.

### Photochromic agent

The topical composition according to the present invention typically contains 0.1 to 5% (w/w) of the photochromic agent. More preferably, the topical composition contains 0.2 to 2% (w/w) most preferably 0.3 to 1.2 %of the photochromic agent.

The one or more photochromic substances in the photochromic agent are non-covalently bound to and/or encapsulated by the aromatic polyether.

Preferably, the one or more photochromic substances are bound to the aromatic polyether. Most preferably, the one or more photochromic substances are bound to the aromatic polyether by pi-stack interaction.

The topical composition of the present invention typically contains the one or more photochromic substances and the aromatic polyether in a molar ratio that lies in the range of 3:1 to 1:4. More preferably, the latter ratio lies in the range of 2:1 to 1:3, most preferably in the range of 3:2 to 1:2.

### Photochromic substances

The topical composition according to the present invention typically contains 0.05-3% (w/w) of the one or more photochromic substances. More preferably, the topical composition contains 0.1-1.4% (w/w) most preferably 0.15-0.6% (w/w) of the one or more photochromic substances.

The one or more photochromic substances employed in the photochromic agent preferably are selected from spiropyrans, spirooxazines, diarylethenes, azobenzenes, spironaphthoxazines, naphthopyranes, and nitrobenzylpyridines combinations thereof. Even more preferably, the one or more photochromic substances are selected from spiropyrans.and spirooxazines. Most preferably, the photochromic substance is a spiropyran. Examples of spiropyrans that can suitably be employed in photochromic agents of the present invention include 1',3',3'-trimethyl-6-nitrospiro[chromene-2,2'-indoline] and 2-(3',3'-dimethyl-6-nitrospiro[chromene-2,2'-indolin]-1'-yl)ethanol combinations thereof.

In accordance with another preferred embodiment, the one or more photochromic substances in the photochromic agent are UV-absorbing substances.

### Aromatic polyether

The topical composition of the present invention typically contains 0.1 to 4% (w/w), more preferably 0.2 to 3% (w/w) and most preferably 0.4 to 1% (w/w) of the aromatic polyether.

The aromatic polyether comprised in the photochromic agent preferably is a dendritic polymer selected from dendrimers, dendrigraft polymers, hyperbranched polymers and combinations thereof. Although the inventors do not wish to be bound by theory it is believed that the one or more photochromic substances are bound very effectively by dendritic polymers due to the presence of multiple 'binding cavities' within these dendritic polymers in which a photochromic substance can be captured and fixated by non-covalent interactions.

According to a particularly preferred embodiment, the aromatic polyether in the photochromic agent is a dendrimer. Even more preferably, the aromatic polyether is a dendrimer selected from G-1 dendrimer, G-2 dendrimer, G-3 dendrimer and combinations thereof. Dendrimers are commonly classified by generation, which refers to the number of repeated branching cycles that are performed during its synthesis. For example, if a dendrimer is made by convergent synthesis, and the branching reactions are performed onto the core molecule three times, the resulting dendrimer is considered a third generation (G-3) dendrimer.

The aromatic polyether in the photochromic agent typically has a molecular weight in the range of 1,000 to 15,000 g/mol. More preferably said molecular weight is in the range of 1,200 to 10,000, most preferably in the range of 1,500 to 5,500 g/mol.

According to another particularly preferred embodiment, the aromatic polyether in the photochromic agents contains a plurality of naphthalene segments. The inventors have found that aromatic polyethers comprising naphthalene segments are particularly effective at improving the photostability of the photochromic substances that are employed in accordance with the present invention.

### Topical compositions

The topical composition of the present invention can be in the form of, for instance, a liquid, a lotion, a cream, a gel, or a toner, and may be applied with an implement or via a face mask, pad or patch. Preferably the composition is in the form of a lotion or gel. A particularly preferred example of such a composition is a leave-on skin lotions. "Skin" as used herein is meant to include skin on the face and body (e.g. neck, chest, back, arms, underarms, hands, legs, buttocks and scalp) and especially to the sun exposed parts thereof. The composition of the invention is also of relevance to applications on any other keratinous substrates of the human body other than skin e.g. hair where products may be formulated with specific aim of providing photoprotection.

Examples of topical compositions according to the present invention are sunscreen formulations, moisturizers, lip balms, antiaging formulations, skin whitening formulations, exfoliating formulations, foundations, powders, eyeshadows, blushes, concealers, lipsticks and glosses, eyeliners and bronzers. Preferably, the topical composition is selected from sunscreen formulations, moisturizers, lip balms, antiaging formulations. sunscreen formulations, moisturizers, lip balms and antiaging formulations. Most preferably, the topical composition is sunscreen formulation.

The topical composition preferably is a non-solid composition. By a non-solid composition is meant a composition that has a critical shear stress (apparent yield stress) of less than 100 Pa, preferably less than 20 Pa at 25°C. The apparent yield stress is preferably at least 5 Pa at 25°C. It is preferred that the non-solid composition has a viscosity at critical shear stress of less 1000 Pa.s at 25°C. Critical shear stress (apparent yield stress) is as defined by H A Barnes (Handbook of Elementary Rheology, University of Wales Aberystwyth, 71-73 (2000)). A preferred non-soild composition is a lotion. A lotion according to the present invention preferably has a viscosity of 1500 to 6000 cP as measured by a Brookefield Viscosity meter using LV # 4, 30 rpm, 30°C.

According to a particularly preferred embodiment, the topical composition is a high sun protection factor (SPF) composition. By high SPF is meant a composition that has an SPF of at least 20, preferably at least 25, more preferably at least 35. This high SPF is preferably achieved using a total amount of organic sunscreens in the range of 0.1 to 15%, preferably from 1 to 10%, more preferably from 2 to 10% by weight of the composition.

The topical composition of the present invention preferably contains 0.1-10 wt.%, more preferably 1-7 wt.% and most preferably 1.5.-5.5 wt.% of UVA sunscreen component.

A preferred UV-A sunscreen component is selected from a dibenzoylmethane, triazine, and benzophenone derivative and combinations thereof. More preferably, the UV-A sunscreen component belongs to the dibenzoylmethane group. Preferred dibenzoylmethane derivatives are selected from 4-tert-butyl-4'-methoxydibenzoylmethane, 2-methyldibenzoylmethane, 4-methyl-dibenzoyl-ethane, 4-isopropyldibenzoyl-methane, 4-tert-butyldibenzoylmethane, 2,4-dimethyldibenzoylmethane, 2,5-dimethyldibenzoylmethane, 4,4'-diisopropyl-dibenzoylmethane, 2-methyl-5-isopropyl-4'-methoxydibenzoylmethane, 2-methyl-5-tert-butyl-4'-methoxy-dibenzoyl methane, 2,4-dimethyl-4'-methoxy dibenzoylmethane or 2,6-dimethyl-4-tert-butyl-4'-methoxy-dibenzoylmethane. The most preferred dibenzoylmethane derivative is 4-tert.-butyl-4'-methoxydibenzoylmethane. Dibenzoylmethane or its derivative is preferably present in 0.2 to 5%, more preferably 0.4 to 3% by weight of the topical composition.

The topical composition of the present invention preferably contains 0.1-10 wt.%, preferably 0.5-7 wt.% and most preferably 1-5 wt.% of UVB sunscreen component.

The UVB sunscreen component is selected from benzophenones, anthranilates, salicylates, cinnamates, camphores, benzylidene malonate; triazone, triazone derivatives, diphenyl acrylates and combinations thereof. More preferably, the UV-B sunscreen component is elected from salicylates, cinnamates, diphyenyl acrylates and combinations thereof. A few of the preferred oil soluble UV-B sunscreens which are commercially available and useful for inclusion in the composition of the invention are Octisalate^{™}, Homosalate^{™}, NeoHelipan^{™}, Octocrylene™, Oxybenzone^{™} or Parsol MCX^{™}.

The topical composition of the invention preferably comprises a cosmetically acceptable base. The cosmetically acceptable base preferably is non-solid. The non-solid base may be thickened or gelled. The cosmetically acceptable base typically constitutes 10 to 99.9%, more preferably 50 to 99% by weight of the topical composition. The cosmetically acceptable base preferably includes water. Water is preferably constitutes 35 to 90%, more preferably 50 to 85%, even more preferably 50 to 80% by weight of the composition

The topical composition may also comprise other diluents. The diluents act as a dispersant or carrier for other materials present in the composition, so as to facilitate their distribution when the composition is applied to the skin. Diluents other than water can include liquid or solid emollients, solvents, humectants, thickeners and powders.

Other ingredients that can suitably be incorporated in the topical composition of the present invention include fragrances, silicone oils, waxes, hydrocarbons, higher fatty acids, essential oils, lipids, skin coolants, vitamins, antioxidants, glycerine, malodour reducing agents, odour controlling materials, softening agents, insect and moth repelling agents, colourants, chelants, bodyfying agents, wrinkle control agents, sanitization agents, skin care agents, glycerine, natural actives, preservatives, chemosensates, (for example menthol), skin lightening agents, sunless-tanning agents (for example dihydroxyacetone), emollients (for example sunflower oil and pertrolatum) and mixtures thereof.

The topical composition preferably comprises 3 to 8 wt.% fatty acids, more preferably C₁₂₋₂₀ fatty acids and even more preferably C₁₄₋₁₈ fatty acids. Most preferably, the fatty acids are selected from palmitic acid, stearic acid and combinations thereof.

Other useful sun-protective agents e.g. inorganic sunscreen may suitably be used in the present topical composition. Examples of inorganic sunscreens include zinc oxide, iron oxide, silica, such as fumed silica, and titanium dioxide. The total amount of inorganic sunscreen that is incorporated in the topical composition preferably is in the range from 1 to 8%, preferably 3 to 8% by weight of the composition.

The topical composition of the invention may additionally comprise a skin lightening agent. The skin lightening agent is preferably chosen from a vitamin B3 compound or its derivative e.g. niacin, nicotinic acid, niacinamide or other well-known skin lightening agents e.g. aloe extract, ammonium lactate, azelaic acid, kojic acid, citrate esters, ellagic acid, glycolic acid, green tea extract, hydroquinone, lemon extract, linoleic acid, magnesium ascorbyl phosphate, vitamins like vitamin B6, vitamin B12, vitamin C, vitamin A, a dicarboxylic acid, resorcinol derivatives (especially 4-susbstituted resorcinols), hydroxycarboxylic acid like lactic acid and their salts e.g. sodium lactate, and mixtures thereof. Vitamin B3 compound or its derivative e.g. niacin, nicotinic acid, niacinamide are the more preferred skin lightening agent as per the invention, most preferred being niacinamide. Niacinamide, when used, is preferably present in an amount in the range of 0.1 to 10%, more preferably 0.2 to 5% by weight of the composition.

Another aspect of the present invention relates to method of providing enhanced sun protection to skin, said method comprising applying a topical composition as defined herein onto the surface of the skin.

Yet another aspect of the present invention relates to a method of preparing a photochromic agent, said method comprising:
(i) providing a solution comprising (i) one or more photochromic substances selected from spiropyrans, spirooxazines, diarylethenes, azobenzenes, spironaphthoxazines, naphthopyranes, nitrobenzylpyridines and combinations thereof; (ii) an aromatic polyether and (iii) organic solvent; and
(ii) removing organic solvent from the solution to precipitate the photochromic agent.

The solution employed in the preparation method preferably contains 1-50 µM of the one or more photochromic substances and 1-500µM, of the aromatic polyether.

The invention is further illustrated by the following non-limiting examples.

### EXAMPLES

### Example 1

### Preparation of aromatic polyether:

2-Bromomethylnaphthalene (0.40 g, 1.79 mmol) in DMF/CH₃CN (1:1, v/v, 10 mL) was added to a ice cooled solution of 3,5-dihydroxybenzyl alcohol (0.10 g, 0.71 mmol) and K₂CO₃ (0.39 g, 1.84 mmol) in DMF/CH₃CN (1:1, v/v, 10 mL). The reaction mixture was stirred for 7 h at 25 °C. The solvent was evaporated to dryness under reduced pressure. The residue was extracted with dichloromethane, washed with water (20 mL x 2), brine (20 mL x 2) and water (20 mL x 2) respectively. The organic phase was dried over Na₂SO₄ and concentrated under reduced pressure. The residue on chromatography with methylene chloride/hexane (3:2, v/v) yielded (0.23 g, 65%) of alkylated benzyl alcohol derivative as a colorless solid. 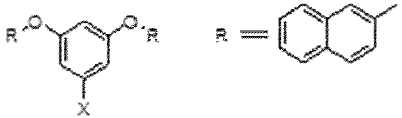 Structure of aromatic polyether, wherein X = CH₂OH

### Preparation of the photochromic compound:

The photochromic spiropyran (1',3'-Dihydro-1',3',3'-trimethyl-6-nitrospiro[2H-1-benzopyran-2,2'-(2H)-indole) used in the study was prepared following the method reported by Wizinger and Wennig. [ref: B. Wizinger and H. Wennig, Helv. Chim. Acta, 23, 247 (1940).

### Photo-irradiation test:

The photochromic compound, spiropyran (10 µM) was treated with the aromatic polyether (100 µM) in a 4:1 (v/v) mixture of ethanol and water. The mixture was allowed to equilibrate in an orbital shaker for at least 30 min at 25°C. Aliquots from the mixture were studied by spectroscopic methods.

The samples were subjected to 100 minutes irradiation with 366 nm UV-light and 200 minutes of visible light (>490 nm light source) in the presence of oxygen. The absorbance of the spiropyran was monitored at 521 nm. The spectral data were compared with those obtained from the controls (only the photochromic compound). Radiation with visible light was done with >490 nm light source

The results are shown in Table 1

**Table 1**

| | Degradation (%) |
|---|---|
| Control (Spiropyran) | 7 |
| Spiropyran + Aromatic ether | 4 |

## Claims

1. A composition for topical application, said composition containing a photochromic agent, said photochromic agent comprising:
(i) one or more photochromic substances selected from spiropyrans, spirooxazines, diarylethenes, azobenzenes, spironaphthoxazines, naphthopyranes, nitrobenzylpyridines and combinations thereof;
(ii) an aromatic polyether;
wherein the one or more photochromic substances are non-covalently bound to and/or encapsulated by the aromatic polyether.

2. Composition according to claim 1, wherein the one or more photochromic substances are selected from spiropyrans and spirooxazines.

3. Composition according to claim 2, wherein the photochromic substance is a spiropyran.

4. Composition according to any one of the preceding claims, wherein the composition contains the one or more photochromic substances and the aromatic polyether in a molar ratio that lies in the range of 3:1 to 1:4.

5. Composition according to any one of the preceding claims, wherein the composition contains 0.1-5% (w/w) of the photochromic agent.

6. Composition according to any one of the preceding claims, wherein the aromatic polyether contains naphthalene segments.

7. Composition according to claim 6, wherein the aromatic polyether is a dendritic polymer selected from dendrimers, dendrigraft polymers, hyperbranched polymers and combinations thereof.

8. Composition according to claim 7, wherein the aromatic polyether is a dendrimer.

9. Composition according to claim 8, wherein the dendrimer is selected from G-1 dendrimer, G-2 dendrimer, G-3 dendrimer and combinations thereof.

10. Composition according to any one of the preceding claims, wherein the composition contains 0.1-10 wt.% of UVA sunscreen component.

11. Composition according to claim 10, wherein the UVA sunscreen component is selected from dibenzoylmethane, triazine, benzophenone derivatives and combinations thereof.

12. Composition according to any one of the preceding claims, wherein the composition contains 0.1-10 wt.% of UVB sunscreen.

13. Composition according to claim 12, wherein the UVB sunscreen component is selected from benzophenones, anthranilates, salicylates, cinnamates, camphores, benzylidene malonate; triazone, triazone derivatives, diphenyl acrylates and combinations thereof.

14. Composition according to any one of the preceding claims, wherein the composition is a liquid, a lotion, a cream, a gel, or a toner,

15. A method of preparing a photochromic agent, said method comprising:
(i) providing a solution comprising (i) one or more photochromic substances selected from spiropyrans, spirooxazines, diarylethenes, azobenzenes, spironaphthoxazines, naphthopyranes, nitrobenzylpyridines and combinations thereof; (ii) an aromatic polyether and (iii) organic solvent; and
(ii) removing solvent from the solution to precipitate the photochromic agent.
